# EUROPÄISCHE PATENTANMELDUNG

(11) **EP 2 037 274 A1**
(43) Veröffentlichungstag der Anmeldung: **18.03.2009**
(21) Anmeldenummer: 07075800.8
(22) Anmeldetag: 11.09.2007
(51) Int. Cl.: G01N 33/68

(54) **Lektinbasierter Glykan-Assay**

(71) Anmelder: GALAB Technologies GmbH, 21502 Geesthacht (DE)
(72) Erfinder: Cartellieri, Simone, 21502 Geesthacht (DE); Umnus, Kirstin, 21502 Geesthacht (DE); Kuballa, Jürgen, 21502 Geesthacht (DE)
(74) Vertreter: Boeckh, Tobias

(57) **Zusammenfassung**

Die Erfindung betrifft die Verwendung von deglykosylierten Detektoren, insbesondere sekundaren Antikorpern, zur Bestimmung der Zuckerstrukturen von Proteinen, insbesondere rekombinanten Proteinen. Sie betrifft weiterhin insbesondere die Verwendung von deglykosylierten Enzymen bei der Bestimmung von Zuckerstrukturen mithilfe einer Enzym-Substrat-Reaktion; des weiteren betrifft die Erfindung ein Verfahren zur Bestimmung der Zuckerstrukturen von Proteinen sowie einen Zuckerbestimmungskit und die Verwendung des Kits zur Bestimmung von Zuckerstrukturen, insbesondere von rekombinanten therapeutischen Proteinen, bevorzugt Immunoglobulinen.

## Beschreibung

Die Erfindung betrifft die Verwendung von deglykosylierten Detektoren, insbesondere sekundären Antikörpern, zur Bestimmung der Zuckerstrukturen von Proteinen, insbesondere rekombinanten Proteinen. Sie betrifft weiterhin insbesondere die Verwendung von deglykosylierten Enzymen bei der Bestimmung von Zuckerstrukturen mithilfe einer Enzym-Substrat-Reaktion; des weiteren betrifft die Erfindung ein Verfahren zur Bestimmung der Zuckerstrukturen von Proteinen sowie einen Zuckerbestimmungskit und die Verwendung des Kits zur Bestimmung von Zuckerstrukturen, insbesondere von rekombinanten therapeutischen Proteinen, bevorzugt Immunoglobulinen.

Rekombinante therapeutische Proteine sind im Stand der Technik bekannt. Im Stand der Technik werden beispielsweise humane Proteine, wie das Erythropoeitin oder Antikörper, rekombinant in CHO-Zellen hergestellt. Diese Technik stellt zwar sicher, dass die in der DNA kodierte Aminosäuresequenz des Proteins reproduziert werden kann. Die posttranslationalen Veränderungen des rekombinanten Proteins, wie die Glykosylierung, sind allerdings in Abhängigkeit vom Zellclon und den Kulturbedingungen variabel. Bei der Bereitstellung von rekombinanten Proteinen, insbesondere therapeutischen Proteinen, ist zu berücksichtigen, dass die Zuckerstrukturen bzw. die Protein-Glykosylierung dazu führt, dass das Protein sich gewebe-, zell-, entwicklungs-, und/oder speziesspezifisch und - typisch in Abhängigkeit von der Glykosylierung verhält. Wesentliche strukturelle Eigenschaften, die eine zentrale Bedeutung beim Einsatz in der Therapie haben, werden durch die Zuckerstrukturen der Proteine determiniert. Bekannte rekombinante therapeutische Proteine sind beispielsweise vollständige, d. h. intakte, monoklonale Antikörper.

Im Stand der Technik ist noch kein Verfahren beschrieben, welches es schnell und automatisierbar ermöglicht, Zuckerstrukturen, insbesondere an rekombinanten therapeutischen Proteinen, wie z. B. intakten monoklonalen Antikörpern, zu bestimmen. Bisher werden im Stand der Technik aufwendige Verfahren wie HPLC, MS oder CE nach Abspaltung der Zucker verwendet. Dazu ist es notwendig, die Zuckerstrukturen mittels chemischer oder enzymatischer Hydrolyse vom Protein zu entfernen. Die enzymatischen Verfahren lassen zwar eine selektive Abspaltung bestimmter Strukturen zu, sind aber in der Durchführung aufwendig und anfällig für Störungen. Im Anschluss an die Abspaltung der Zucker muss eine Abtrennung vom Protein erfolgen, die häufig säulenchromatographisch erfolgt. Diese Vorbereitungschritte und die oftmals geringe Empfindlichkeit der anschließenden Analyseverfahren führen dazu, dass eine nicht geringe Menge des rekombinanten Proteins (500-1000 µg) benötigt wird, was in der Praxis einen Nachteil darstellt. Die Bestimmung der Zuckerstrukturen erfordert nicht selten die Kombination mehrerer analytischer Verfahren, wie zum Beispiel eine massenspektrometrische Untersuchung mittels MALDI-MS, welche qualitative Ergebnisse liefert und zwischen Hexosen nicht differenzieren kann, und eine quantitative Bestimmung definierter Zucker mittels HPLC oder photometrischer Verfahren. Der Einsatz der letztgenannten Verfahren ist vom Vorhandensein reiner Glykane als Vergleichsstandards abhängig.

Der Stand der Technik offenbart unter anderem eine Methode zur Detektion des terminalen Glykosylierungsmusters von IgG. Hierzu wird eine Probe, die das Protein, welches untersucht werden soll, umfasst, auf Nitrocellulose immobilisiert. Anschließend werden die Proteine, bevorzugt IgG, so behandelt, dass die Zuckerreste (Gal, GlcNAc) freigesetzt werden. Die Detektion der IgGs in der Probe erfolgt mit markierten Lektinen, wobei die Markierung wiederum mit Enzymen erfolgt (WO 89/04490).

Eine weitere Methode des Standes der Technik umfasst die Immobilisierung von Lektinen auf einer Platte und die Detektion mit sekundärem Antikörper-Enzym-Konjugat. Das Lektin ist hierbei so ausgewählt, dass es nur eine abnorme Zuckerstruktur erkennen kann; es ist daher nicht geeignet, IgGs mit Galactose, Fukose und anderen Zuckern zu bestimmen (WO 98/16825).

Weiterhin ist im Stand der Technik eine Methode zur Detektion eines Glykoproteins mit markierten Lektinen beschrieben, bei der zunächst ein sogenannter Fänger-Antikörper (Fab-Teil) immobilisiert wird. Dessen Fc-Teil wird abgespalten, um Zucker, die den Messvorgang behindern können, zu verwerfen. Nachteilhafterweise wird in diesem Verfahren (WO 03/087821) das Problem der Fab-Glykosylierung nicht gelöst und weiterhin die Glykosylierung des Enzyms bei der Bestimmung der Zuckerstrukturen nicht berücksichtigt.

In der EP 0 399 464 A2 werden fünf Methoden offenbart, bei denen spezifische Zuckerreste mithilfe von Kunststoffpartikeln bestimmt werden. Diese Methode erlaubt wegen möglicher Zuckerstrukturen an den eingesetzten Substanzen keine zufriedenstellenden Ergebnisse, ebenso wie das Verfahren gemäß EP 0 662 611 A2, bei welchem Glykokonjugate im Sandwichaufbau mit Lektinen untersucht werden, indem zunächst ein Fänger-Antikörper immobilisiert wird, der chemisch behandelt vorliegt, um den Zucker zu inaktivieren und falsche Hintergrundsignale zu vermeiden.

Alle Methoden des Standes der Technik sind nicht geeignet, Zuckerstrukturen vor allem bei rekombinanten Proteinen wie Immoglobulinen, insbesondere IgG sicher bzw. effektiv zu bestimmen.

Im Lichte des Standes der Technik war es völlig überraschend, dass deglykosylierte Detektoren, insbesondere sekundäre Antikörper, verwendet werden können, um Zuckerstrukturen von rekombinanten Proteinen wie Immunoglobulinen (primären Antikörpern) sicher und effektiv zu bestimmen. Insbesondere war es überraschend, dass bei Verwendung einer Enzym-Substrat-Reaktion zur Bestimmung der Zuckerstrukturen von rekombinanten Proteinen überraschend gute Resultate erzielt werden, wenn neben dem Detektor wie dem sekundären Nachweisantikörper auch das Enzym deglykosyliert vorliegt.

Die Erfindung betrifft demgemäß die überraschende Lehre, bei der Bestimmung der Zuckerstrukturen von insbesondere rekombinanten Proteinen deglykosylierte Detektoren zur Bestimmung der Zuckerstrukturen einzusetzen, wobei die Detektoren bevorzugt sekundäre Antikörper, Protein A und/oder Fragmente hiervon sind und die rekombinanten Proteine Erkennungsmoleküle, bevorzugt ausgewählt aus der Gruppe der IgG-, IgM-, IgE-, IgD-, IgA-, IgY- und/oder IgB-Antikörper.

Für den Fachmann mit durchschnittlichem Können sind die einzelnen technischen Merkmale der erfindungsgemäßen Lehre ausreichend klar definiert. Ihm ist aus der ELISA-Technik bekannt, dass er rekombinante Proteine, bevorzugt in Form von Erkennungsmolekülen, besonders bevorzugt in Form von Antikörpern, sehr gut und sicher mit Detektoren nachweisen kann, wenn diese Detektoren spezifisch mit den bevorzugten Antikörpern reagieren. Es kann sich bei den Detektoren um Lektine, aber insbesondere um sekundäre Antikörper oder Fragmente hiervon handeln.

Dem Fachmann sind Detektionsmethoden für die Sichtbarmachung von primären Antikörpern bekannt. Häufig werden für den Nachweis der primären Antikörper sekundäre Antikörper eingesetzt. Selbstverständlich ist es möglich, dass der Fachmann andere spezifische Detektoren für die primären Antikörper einsetzt, wie beispielsweise Protein A. Protein A ist im Sinne der Erfindung ein Protein von 40 bis 60 kDa, das ursprünglich aus der Zellwand des Bakteriums Staphylococcus aureus stammt. Protein A wird vorteilhafterweise eingesetzt, um Immunoglobuline (primäre Antikörper) zu binden. Es werden Proteine verschiedener Säugerarten gebunden, vor allem IgG. Protein A bindet an die Fc-Region der Immunoglobuline durch Interaktion mit der schweren Kette. Insbesondere bindet Protein A mit hoher Affinität an humanes IgG1 und IgG2 sowie IgG2a und IgG2b. Aber auch IgM, IgA und IgG und rekombinante monoklonale Antikörper aus nicht-humanen Expressionssystemen werden spezifisch gebunden. Wenn der Fachmann vor die Aufgabe gestellt ist, primäre Antikörper zu binden, so weiß er, dass er neben sekundären Antikörpern, Protein A und/oder Fragmenten hiervon auch andere Detektoren verwenden kann. wobei Detektoren im Sinne der Erfindung Moleküle sind, die mit den primären Antikörpern spezifisch interagieren. Bei dem Detektor im Sinne der Erfindung kann es sich daher bevorzugt auch um Protein G handeln. Besonders spezifisch reagiert Protein G mit Ig1, Ig2 und Ig4 von humanem IgG; weiterhin bindet Protein G auch an Ig3 und weitere therapeutische Proteine. Überraschend ist es, dass Zuckerstrukturen von bevorzugt primären Antikörpern mit Detektoren sehr gut bestimmt werden können, wenn die Detektoren deglykosyliert vorliegen. Die Deglykosylierung kann vorteilhafterweise mithilfe von Glykosidasen, wie den Endo- und Exoglycosidasen erfolgen. Hier stehen dem Fachmann im Stand der Technik eine Reihe von Enzymen zur Verfügung, die im Zusammenhang mit der Erfindung ein überraschend gutes Resultat zeigen, wie z.B. die N-Glykanasen, O-Glykanasen, Endoglykosidasen-H, F1, F2, F3, und die Sialidasen, Galaktosidasen, Mannosidasen und Fukosidasen. Ebenso stehen Methoden der chemischen Deglykosylierung zur Verfügung, wie die Hydrazinolyse oder milde Säurehydrolyse zur Abspaltung von Sialinsäuren.

Neben den sekundären Antikörpern oder Protein A und G, die zum Nachweis der primären Antikörper/therapeutischen Proteine eingesetzt werden können, ist es auch möglich, Fragmente dieser einzusetzen. Der Fachmann kann, ohne selbst erfinderisch tätig zu werden, durch einfache Routineversuche eruieren, ob ein Fragment eines sekundären Antikörpers oder von Protein A oder G noch eingesetzt werden kann, um Zuckerstrukturen von rekombinanten Proteinen, bevorzugt Antikörpern zu bestimmen.

In einer bevorzugten Ausführungsform erfolgt die Bestimmung der Zuckerstrukturen mithilfe einer Enzym-Substrat-Reaktion, wobei das Enzym neben dem eingesetzten Detektor ebenfalls deglykosyliert ist. Enzym-Substrat-Reaktionen sind bekannte Verfahren zum Nachweis von Antikörpern. Enzym-Substrat-Reaktionen sind häufiger Bestandteil der indirekten Methode des Nachweises eines spezifischen Antikörpers (Primärantikörpers). Ein bekanntes Verfahren der Enzym-Substrat-Reaktion sieht vor, dass ein Sekundärantikörper mit einem Enzym gekoppelt vorliegt, welches mit einem Substrat/Chromogen eine Farbreaktion initiiert, die durch Messgeräte oder durch einfaches Betrachten detektiert werden kann. Häufig eingesetzte Enzyme sind die Meerrettich-Peroxidase mit den Substraten ABTS, OPD oder TMB, die bovine alkalische Phosphatase mit den Substraten PNPP, FDP, NBT, oder auch die Enzyme beta-Galaktosidase und Glucose Oxidase mit entsprechenden Substraten. Bei der Verwendung der alkalischen Phosphatase werden als Substrate Phosphatverbindungen eingesetzt, von denen die alkalische Phosphatase Phosphat abspaltet, wodurch die freigesetzte Verbindung zu einem farbigen Endprodukt reagieren kann. Beispielsweise liefert Neufuchsin ein rosa/rotes Reaktionsprodukt, Naphthol-HS-MX-Phosphat bildet einen roten Farbstoff und 5-Brom-4-chlor-3-indoxylphosphat wird in Verbindungen mit Nitroblau-Tetrazoliumchlorid zu einem violetten bis blauen Farbstoff umgesetzt. Para-Nitrophenylphosphat (PNPP) wird zu einem gelben Farbstoff umgesetzt, der bei 405 nm detektiert werden kann, und Fluorescein-Diphosphat (FDP) wird nach Umsetzung zu Fluorescein mittels Fluoreszenzmessung detektiert.

Die erfindungsgemäße Verwendung der deglykosylierten Detektoren - bevorzugt sekundäre Antikörper - oder Enzyme erfolgt insbesondere bei einem Verfahren, welches die folgenden Schritte umfasst:
a) Bereitstellung des zu untersuchenden Proteins, bevorzugt rekombinanten Proteins, insbesondere eines rekombinanten therapeutischen Glykoproteins;
b) Inkubation mit einem markierten Detektor, bevorzugt einem sekundären Antikörper oder einem Protein A oder Fragmenten davon, welches deglykosyliert ist; und
c) Bestimmung der Interaktion zwischen dem Protein und dem Detektor.

In diesem Verfahren können insbesondere Immunoglobuline, ausgewählt aus der Gruppe IgG, IgM, IgE, IgD, IgA, IgY und/oder IgW in Bezug auf ihre Zuckerstrukturen untersucht werden. D. h. die erfindungsgemäße Verwendung von deglykosylierten Detektoren und/oder Enzymen - bevorzugt Detektoren und Enzymen - ermöglicht effiziente, sichere, schnelle und automatisierbare Verfahren zur Bestimmung der Zuckerstrukturen von Proteinen, insbesondere Immunoglobulinen.

Bei dem Verfahren kann bevorzugt zunächst ein Array von Lektinen auf eine Mikrotiterplatte aufgebracht werden, die Zuckerstrukturen am IgG erkennen. Lektine sind im Stand der Technik als Proteine bekannt, die ein oder mehrere Bindungsstellen für Zuckerstrukturen aufweisen. Lektine werden daher eingesetzt, um Zuckerstrukturen zu detektieren oder abzutrennen. Die Lektine können native Lektine sein ausgewählt aus den Organismen Agaricus bisporus, Aleuria aurantia, Amaranthus caudatus, Anguilla anguilla, Arachis hypogaea, Artocarpus integrifolia, Griffonia (Bandeiraea) simplicifolia I, Bauhinia purpurea alba, Cicer arietinum, Codium fragile, Canavalia ensiformis, Datura stramonium, Dolichos biflorus, Erythrina coralldendron, Erythrina cristagalli, Euonymos europaeus, Galanthus nivalis, Glycine max, Helix aspersa, Helix pomatia, Hippeastrum hybrid, Lens culinaris, Limulus polyphemus, Lotus tetragonolobus, Lycopersicon esculentum, Maackia amurensis, Maclura pomifera, Narcissus pseudonarcissus, Phaseolus coccineus, Phaseolus limensis, Phaseolus vulgaris, Phytolacca americana, Pisum sativum, Phosphocarpus tetragonolobus, Ricinus communis, Sambucus nigra, Solanum tuberosum, Sophora japonica, Triticum vulgaris, Tritrichomonas mobilensis (Protozoe), Ulex europaeus, Vicia faba, Vicia villosa, Viscum album, Wisteria floribunda und/oder auch rekombinante Lektine. In einer Ausführungsform des Verfahrens werden unterschiedliche Lektine (mindestens eines) auf der Oberfläche einer Mikrotiterplatte immobilisiert. Die Lektine werden so gewählt, dass sie die Zuckerstrukturen am IgG erkennen, insbesondere N-Acetylglucosamin, Galaktose, Mannose, Fukose und Sialinsäure (N-Acetlyneuraminsäure). Die Erkennung einer spezifischen Zuckerstruktur am IgG führt dazu, dass das IgG Molekül an der Oberfläche zurückgehalten wird, während andere IgG-Moleküle, die diese Struktur nicht enthalten, abgewaschen werden.

Als Kontrolle für die IgGs kann Protein A oder Fragmente dessen eingesetzt werden. Protein A bindet an einem nicht-glykosylierten Teil des IgG (Fc) und stellt daher ein Referenzsignal zur Verfügung, das im Verhältnis zur Gesamtmenge des im Verfahren eingesetzten IgG steht. Das Protein A Signal kann also zur Kontrolle des Verfahrens und zur Kalibrierung verwendet werden, um aus dem Verfahren auch quantitative Informationen zu erhalten. Die Einzelergebnisse für die Zuckerstrukturen können im Vergleich zu der Protein A-Kontrolle auf den IgG-Gesamtgehalt bezogen werden.

Nachdem in der bevorzugten Ausführungsform der Array von Lektinen auf einer Mikrotiterplatte bereitgestellt wurde, kann dieser Array mit der Probe, die die zu untersuchenden Glykoproteine umfasst, in Kontakt gebracht werden. Hierbei sind die zu untersuchenden Glykoproteine insbesondere rekombinante Antikörper; die als primäre Antikörper bezeichnet werden. Primäre Antikörper sind die Moleküle, deren Zuckerstruktur bestimmt wird, indem sie bevorzugt mit einem zweiten Antikörper (= sekundärer Antikörper) detektiert werden. Vorteilhafterweise kann die Probe ohne weitere Vorbereitung, wie beispielsweise einer Markierung der in ihr enthaltenen Glykoproteine, eingesetzt werden. Im Stand der Technik sind aufwändige Vorbereitungen der Probe erforderlich.

Nach diesem Verfahrensschritt liegt ein Array von Lektinen auf einer Mikrotiterplatte vor, an dem die spezifisch bindenden, zu untersuchenden Glykoproteine gebunden vorliegen.

Die Interaktion zwischen dem zu untersuchenden Protein und dem Lektin kann beispielsweise mit einer dem Fachmann bekannten Enzym-Substrat-Reaktion bestimmt werden, wie sie dem Fachmann insbesondere von ELISA-Tests bekannt ist.

Im Sinne der Erfindung wird die Interaktion mit einem so genannten Detektor bestimmt, indem der Detektor das zu untersuchende Glykoprotein in der Probe spezifisch erkennt und zwar besonders bevorzugt an einer Stelle, die nicht das Glykan ist. Der Detektor bzw. das Detektormolekül ist insbesondere ein sekundärer Antikörper oder ein Fragment dessen, es kann sich aber auch um ein Protein A oder ein Fragment hiervon handeln.

Es ist erfindungswesentlich, dass diese Detektormoleküle deglykosyliert vorliegen, um falsch-positive Hintergrundsignale ausschließen zu können. Experimente haben gezeigt, dass man bei Verwendung von glykosylierten Detektoren ein Mischergebnis erhält, dass sowohl von den Zuckerstrukturen des Detektors als auch von denen des zu analysierenden Proteins bestimmt wird. Das Anwendungsbeispiel 1 zeigt die Signale eines glykosylierten Detektors auf 2 unterschiedlichen Lektinen.

Überraschenderweise führt auch die Deglykosylierung des Enzyms, welches bei der Enzym-Substrat-Reaktion verwendet wird, zu einem so überraschend guten Ausschluss der falsch-positiven Hintergrundsignale, dass eine einfache, sichere und effiziente Bestimmung der Zuckerstrukturen der rekombinanten therapeutischen Proteine möglich ist. Experimente haben vielmehr gezeigt, dass man bei Verwendung von glykosylierten Enzymen ein Mischergebnis erhält, dass sowohl von den Zuckerstrukturen des Enzyms als auch von denen des zu analysierenden Proteins bestimmt wird. Das Anwendungsbeispiel 2 zeigt den Vergleich eines glykosylierten und eines nicht-glykosylierten Enzym auf einer lektinbeschichteten Mikrotiterplatte.

In einer bevorzugten Ausführungsform der Erfindung erfolgt die Bestimmung der Interaktion zwischen dem Protein und dem Detektor über eine Enzym-Substrat-Reaktion.

In einer weiteren bevorzugten Ausführungsform der Erfindung liegt das rekombinante Protein immobilisiert vor. Bei der Immobilisierung handelt es sich im Sinne der Erfindung um verschiedene Verfahren zum Fixieren der rekombinanten Proteine, insbesondere der Antikörper, insbesondere von IgG. Die Fixierung insbesondere der Antikörper kann durch biologische, chemische oder physikalische Einwirkung erfolgen. Beispielsweise ist es möglich, die rekombinanten Proteine/Antikörper trägergebunden zu fixieren, oder aber durch Quervernetzung, durch Einschluss oder durch das Einbringen in Mikrokapseln. Bei der Bindung an einen Träger (z. B. Mikrotiterplatte) erfolgt die Fixierung durch Adsorption, Ionenbindung oder kovalente Bindung. Beispielsweise können die rekombinanten Proteine/Antikörper auch durch einen immobilisierten Sekundärantikörper (Fängerantikörper) oder Protein A an der Oberfläche fixiert werden. Bevorzugt wird das Protein durch die Fixierung in seiner Zuckerstruktur nicht nachteilhaft modifiziert. Bei dem Einschluss in eine semipermeable Membran erfolgt die Fixierung in Form des Einschlusses in Gele, Mikrokapseln oder Fasern.

Bevorzugt erfolgt die Fixierung des rekombinanten Proteins, insbesondere der Antikörper, bevorzugt von IgG, an eine Mikrotiterplatte. Bei Mikrotiterplatten handelt es sich bevorzugt um Plastikplatten in der Regel aus Polystyrolen oder Polyvinylchlorid, die für unterschiedliche immunologische Arbeitsgänge eingesetzt werden können. Die gängigen Mikrotiterplatten haben 96 Vertiefungen mit einem Fassungsvermögen von je 250 Mikroliter. Selbstverständlich ist es auch möglich, Mikrotiterplatten aus anderen Materialien einzusetzen oder aber die Anzahl der Vertiefungen zu variieren. Für den Hochdurchsatz sind beispielsweise auch Mikrotiterplatten mit 386 oder 1544 Vertiefungen vorteilhaft.

In einer besonders bevorzugten Ausführungsform der Erfindung wird das rekombinante Protein, insbesondere der Antikörper, bevorzugt IgG über ein Lektin fixiert/immobilisiert. Lektine im Sinne der Erfindung sind insbesondere komplexe Proteine, die spezifische Kohlenhydratstrukturen des Proteins, welches untersucht werden soll, binden und dadurch in der Lage sind, dieses zu fixieren. Lektine werden insbesondere aus Pflanzenarten, vor allem aus Hülsenfrüchten, aber auch aus tierischen Organismen und Zellen gewonnen. Bevorzugte Lektine haben eine Spezifität für Galaktose, Mannose, N-Acetylglukosamin, Fukose und Sialinsäuren (N-Acetylneuraminsäure) und werden beispielsweise aus der Schwertbohne, Gartenbohne, Ricinusbohne, Sojabohne, Linse, Erbse, Erdnuss, Weizen, Kartoffel, Schneeglöckchen, dem schwarzen Holunder, dem Amur-Gelbholz, dem Stechginster, dem Pfaffenhütchen, und/oder aus Pilzen wie dem O-rangebecherling gewonnen.

In einer besonders bevorzugten Ausführungsform erfolgt die Immobilisierung des zu untersuchenden therapeutischen rekombinanten Glykoproteins, insbesondere des primären Antikörpers dergestalt, dass es über ein Lektin fixiert wird, welches an einer Mikrotiterplatte immobilisiert vorliegt. Dem Fachmann sind Kombinationen aus Mikrotiterplatte und einem hierauf immobilisierten Lektin aus anderen Verwendungen bekannt. Derartige Kombinationen sind beispielsweise aus Enzym-Immunoassays bekannt. Enzym-Immunoassays umfassen Detektionssysteme aus Enzymen und Substraten bzw. Radioisotopen. Der Begriff der Detektionssysteme ist ein im Stand der Technik üblicher Begriff und lexikalisch nachweisbar und muss daher im Detail nicht erläutert werden; wie auch der Begriff des Detektors. Die Immobilisierung eines Lektins auf einer Mikrotiterplatte ist bevorzugter Bestandteil der bekannten Festphasentechnik, beispielsweise der Affinitätschromatographie.

Bevorzugt werden Lektine auf der Mikrotiterplatte immobilisiert, die mit einer Zuckerstruktur des rekombinanten Proteins, insbesondere der Antikörper, bevorzugt IgG spezifisch wechselwirken. Eine spezifische Wechselwirkung ist eine Wechselwirkung, die einen Rückschluss auf eine Zuckerstruktur des rekombinanten Proteins zulässt. Eine Wechselwirkung, die dergestalt erfolgt, dass die Zuckerstruktur nicht bestimmt werden kann, ist nicht spezifisch im Sinne der Erfindung. Eine nicht spezifische Wechselwirkung wäre beispielsweise eine Wechselwirkung eines Mittels mit der Mikrotiterplatte, mit dem Lektin und/oder dem rekombinanten Protein, insbesondere seiner Zuckerstruktur, so dass keine Bestimmung der Zuckerstruktur möglich ist.

Besonders bevorzugt ist es, dass in vorteilhaften Ausführungsformen der Erfindung das rekombinante Protein/Glykoprotein ohne eine Markierung eingesetzt wird. D. h., vorteilhafterweise ist es nicht erforderlich, dass die Probe, die das zu untersuchende Glykoprotein/rekombinante Protein, bevorzugt den Antikörper, insbesondere IgG umfasst, vorbereitet wird, beispielsweise indem eine Markierung mit Enzymen, Biotin/Streptavidin, Fluoreszenzfarbstoffen wie FITC oder Isotopen erfolgt.

In einer besonders bevorzugten Ausführungsform umfasst das erfindungsgemäße Verfahren die folgenden Schritte:
a) Bindung mindestens eines Lektins auf einer Mikrotiterplatte;
b) In-Kontakt-Bringen des Lektins mit dem zu untersuchenden rekombinanten Protein/Glykoprotein;
c) Inkubieren mit einem markierten Detektor, umfassend einen sekundären Antikörper, Protein A und/oder Fragmenten dieser;
d) Nachweis des Detektors mit Hilfe einer Enzym-Substrat-Reaktion, wobei der Detektor und das Enzym deglykosyliert sind.

Selbstverständlich ist es auch möglich, das erfindungsgemäße wie folgt zu modifizieren, so dass es folgende chronologische Schrittabfolge umfasst:
a) Bindung mindestens eines deglykosylierten Detektors bevorzugt eines sekundären Antikörpers auf einer Mikrotiterplatte;
b) In-Kontakt-Bringen des Detektors mit dem zu untersuchenden Protein/Glykoprotein;
c) Inkubieren mit einem markierten Lektin;
d) Nachweis des Detektors mit Hilfe einer Enzym-Substrat-Reaktion, wobei der Detektor und das Enzym deglykosyliert sind.

Besonders bevorzugt ist die vollständige Deglykosylierung im Zusammenhang mit den erfindungsgemäßen Verwendungen des deglykosylierten Detektors bzw. des Enzyms oder der Fragmente. Die vollständige Deglykosylierung des Detektors bzw. dessen Fragmentes wird z. B. durch die enzymatische Spaltung des Detektors erreicht. Anschließend wird das nicht glykosylierte Fab-Fragment oder Fab-2-Fragment verwendet, wenn der Detektor ein sekundärer Antikörper ist. Nicht-glykosylierte Fab-Fragmente erhält man entweder durch Auswahl aus nativen, polyklonalen Formen oder durch die rekombinante, monoklonale Herstellung eines sekundären Antikörpers oder Fab-Fragmentes nach Ausschalten der möglichen Glykosylierungsstellen am Fab-Teil. Dem Fachmann sind diese Methoden bekannt. Bevorzugte Enyzme für die enzymatische Spaltung des Detektors, insbesondere des sekundären Antikörpers sind Papain und Pepsin. Weiterhin ist es vorteilhaft, eine vollständige oder teilweise Deglykosylierung des Detektors mit einer Behandlung dessen mit zuckerabspaltenden Enzymen, bevorzugt Glykosidasen vorzunehmen. Hierzu können bevorzugt folgende Enzyme verwendet werden: Endo H F1, Endo H F2, Endo H F3, PNGase F, N-Glycanase, Galaktosidase, Mannosidase, Sialidase und/oder Fukosidase. Auf diese Weise können sowohl der vollständige Detektor/Sekundärantikörper oder die Fragmente dessen (Fc, Fab, Fab-2) behandelt. d. h. degly-kosyliert werden. Eine weitere bevorzugte Variante der Deglykosylierung ist eine chemische Behandlung, beispielsweise mit Periodat. Es kann weiterhin vorteilhaft sein, den Sekundärantikörper aus Expressionssystemen zu verwenden, die nur definierte Zuckerstrukturen synthetisieren, wie z. B. high-mannose-type, in Hefezellen oder ausgewählten, modifizierten eukaryotischen Systemen. Eine weitere bevorzugte Variante ist die Verwendung eines Detektors (z.B. Protein A) aus Expressionssystemen, die nicht zur post-translationalen Modifikation fähig sind, wie z. B. prokaryotische Systeme.

Bei der Bereitstellung des deglykosylierten Enzyms ist es bevorzugt möglich, dieses rekombinant oder nativ in prokaryotischen Expressionssystemen herzustellen, so dass es nicht glykosyliert ist. Dies ist beispielsweise besonders bevorzugt mit der nativen alkalischen Phosphatase oder der rekombinanten alkalischen Phosphatase aus E. coli möglich. Die vollständige oder teilweise enzymatische Deglykosylierung des Enzyms kann außerdem mit folgenden Enzymen erfolgen: Endo H F1, Endo H F2, Endo H F3, N-Glycanase, Galaktosidase, Mannosidase, Sialidase und/oder Fukosidase. Weiterhin ist die Verwendung eines Enzyms aus Expressionssystemen möglich, die nur definierte Zuckerstrukturen synthetisieren (z. B. high-mannose-type, in Hefezellen oder andere ausgewählte eukaryotische Systeme).

In einer weiteren bevorzugten Ausführungsform der Erfindung wird das rekombinante Protein über ein Lektin immobilisiert.

In einer weiteren bevorzugten Ausführungsform der Erfindung liegt das Lektin auf einer Mikrotiterplatte immobilisiert vor.

In einer weiteren bevorzugten Ausführungsform der Erfindung sind die Lektine so ausgewählt, dass sie mit einer Zuckerstruktur des rekombinanten Proteins spezifisch wechselwirken.

Im Folgenden soll die Erfindung anhand von Beispielen näher erläutert werden, ohne auf dieses beschränkt zu sein (Anwendungsbeispiele 1 bis 3).

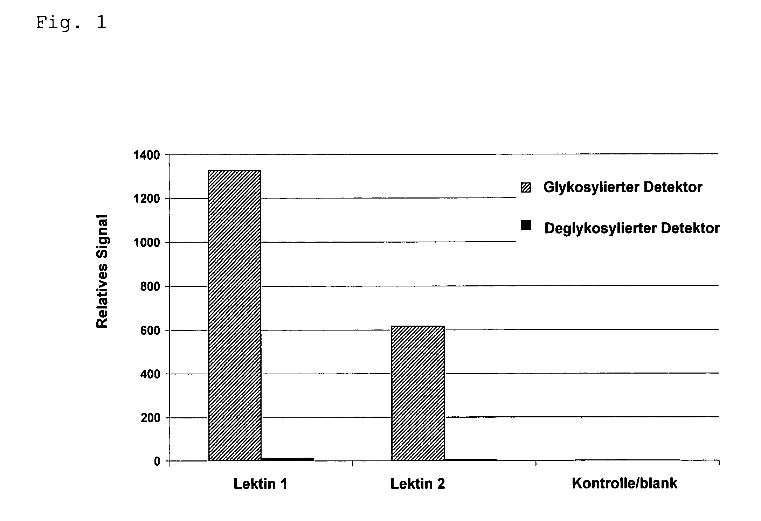

### Anwendungsbeispiel 1 (Fig. 1):

Die Oberfläche einer Mikrotiterplatte wurde mit den Lektinen Concanavalin A (1) und Sambucus nigra agglutinin (2) beschichtet. Um zu ermitteln, welches Signal von einem glykosylierten Detektor herrührt, wurde ein nativer Sekundärantikörper (aus Ziege) ohne Zugabe einer Probe direkt vermessen. Als Vergleich wurde ein deglykosylierter Detektor (ProteinA) vermessen.

Die Ergebnisse zeigen hohe Signale des glykosylierten Sekundärantikörpers (Detektors) auf beiden verwendeten Lektinen. Die Kontrolloberfläche (Blank = serum albumin) zeigt erwartungsgemäß keine Anbindung des glykosylierten Detektors. Der deglykosylierte Detektor Protein A liefert ebenfalls keine Signale auf den Lektinen.

Das Anwendungsbeispiel zeigt, dass nur die Verwendung eines deglykosylierten Detektors falsch-positive Hintergrundsignale ausschließen kann.

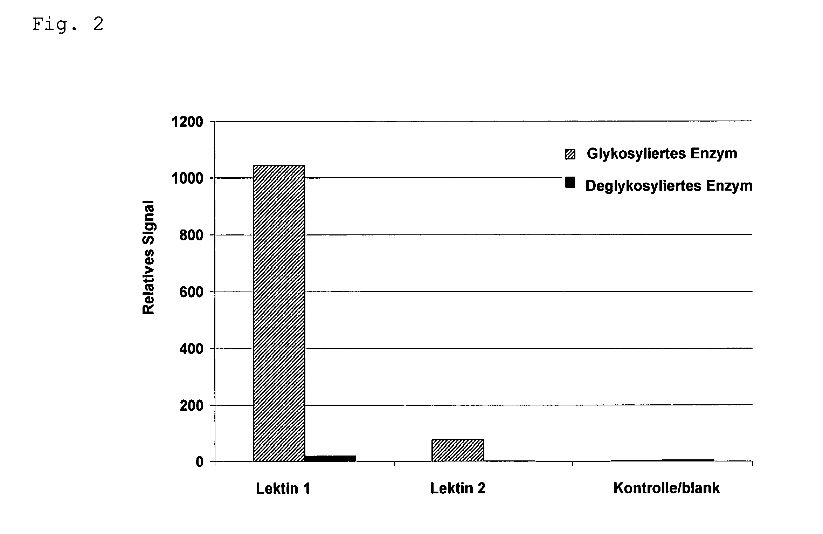

### Anwendungsbeispiel 2 (Fig. 2):

Die Oberfläche einer Mikrotiterplatte wurde mit den Lektinen Aleuria aurantia lektin (1) und Galanthus nivalis agglutinin (2), Protein A und Serumalbumin (blank) beschichtet. Um zu ermitteln, welches Signal von einem glykosylierten Enzym herrührt, wurden im Vergleich ein glykosyliertes und ein deglykosyliertes Enzym (hier alkalische Phosphatase) vermessen. Zur Verdeutlichung wurde in diesem Fall keine Probe, z.B. ein therapeutisches IgG, zugegeben.

Die Ergebnisse zeigen ein hohes Signal des glykosylierten Enzyms am Lektin, das deglykosylierte Enzym zeigt hingegen kein Signal. Die Kontrolloberflächen Protein A und Serumalbumin zeigen erwartungsgemäß keine Anbindung des glykosylierten und des deglykosylierten Enzyms.

Das Anwendungsbeispiel zeigt, dass nur die Verwendung eines deglykosylierten Enzyms die falsch-positiven Hintergrundsignale ausschließen kann.

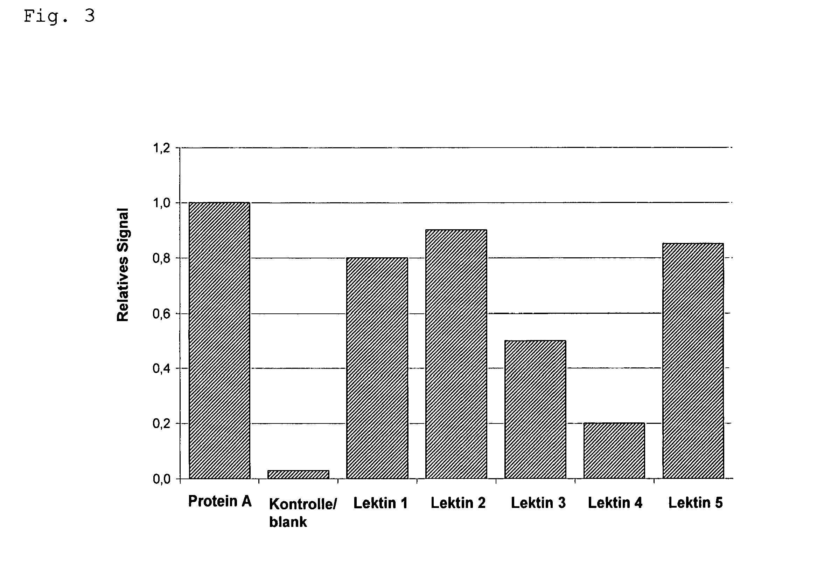

### Anwendungsbeispiel 3 (Fig. 3):

Die Oberfläche einer Mikrotiterplatte wurde mit einem Array von Lektinen (1-5), Protein A als Kontrolle und Serumalbumin (als blank) beschichtet. Die untersuchte Probe, ein rekombinantes, therapeutisches IgG, wurde anschließnd aufgegeben und bindet entsprechend der vorhandenen Zuckerstrukturen an den Lektinen. Zur Detektion wurde ein biotinylierter Detektor (deglykosyliertes Fab-Fragment eines Sekundärantikörpers) eingesetzt, der nach einem Waschschritt mittels Spreptavidin-Alkalischer Phosphatase (deglykosyliert, rekombinant aus Ecoli) und dem Substrat Para-nitrophenylphosphat ausgelesen wird.

Die Ergebnisse zeigen unterschiedliche hohe Signale für die verschiedenen Lektine, die auf die speziellen Zuckerstrukturen der Probe zurückzuführen sind. Das ProteinA-Signal dient dabei der Normierung, damit unterschiedliche Proben direkt verglichen werden können. Das Anwendungsbeispiel zeigt, dass der erfindungsgemäße Assay geeignet ist, die Zuckerstrukturen von rekombinanten Proteinen schnell, automatisierbar und störungsfrei zu analysieren.

## Patentansprüche

1. Verwendung von deglykosylierten Detektoren zur Bestimmung der Zuckerstrukturen von Proteinen, insbesondere rekombinanten Proteinen, wobei die Detektoren bevorzugt sekundäre Antikörper, Protein A und/oder Fragmente hiervon sind und die bevorzugt rekombinanten Proteine Erkennungsmoleküle, bevorzugt primäre Antikörper, sind, ausgewählt aus der Gruppe umfassend IgM, IgE, IgD, IgA, IgY und/oder IgW, bevorzugt IgG.

2. Verwendung nach dem vorhergehenden Anspruch,
**dadurch gekennzeichnet, dass**
die Bestimmung der Zuckerstrukturen mit Hilfe einer Enzym-Substrat-Reaktion erfolgt, wobei das Enzym deglykosyliert ist.

3. Verwendung nach einem der vorhergehenden Ansprüche,
**dadurch gekennzeichnet, dass**
die Deglykosylierung durch Abspaltung der Zucker vom Enzym, Abtrennung glykosylierter Molekülteile und/oder der Herstellung des Enzyms in einem bakteriellen Expressionssystem erfolgt.

4. Verwendung nach einem der vorhergehenden Ansprüche,
**dadurch gekennzeichnet, dass**
die Deglykosylierung des Detektors durch Abspaltung der Zucker vom Detektor, Abtrennung glykosylierter Molekülteile und/oder durch die rekombinante Herstellung des deglykosylierten Detektors erfolgt.

5. Verfahren zur Bestimmung der Zuckerstrukturen von rekombinanten Proteinen, insbesondere Immunoglobulinen,
**dadurch gekennzeichnet, dass**
das Verfahren folgende Schritte umfasst:
a) Bereitstellung des zu untersuchenden rekombinanten Proteins, insbesondere eines rekombinanten therapeutischen Glykoproteins;
b) Inkubation mit einem markierten Detektor, bevorzugt einem sekundären Antikörper oder einem Protein A oder Fragment davon, welches deglykosyliert ist; und
c) Bestimmung der Interaktion zwischen dem Protein und dem Detektor.

6. Verfahren nach dem vorhergehenden Anspruch,
**dadurch gekennzeichnet, dass**
die Bestimmung der Interaktion zwischen dem Protein und dem Detektor über eine Enzym-Substrat-Reaktion bestimmt wird.

7. Verfahren nach einem der vorhergehenden Ansprüche,
**dadurch gekennzeichnet, dass**
das rekombinante Protein immobilisiert vorliegt.

8. Verfahren nach dem vorhergehenden Anspruch,
**dadurch gekennzeichnet, dass**
das rekombinante Protein über ein Lektin immobilisiert wird.

9. Verfahren nach dem vorhergehenden Anspruch,
**dadurch gekennzeichnet, dass**
das Lektin auf einer Mikrotiterplatte immobilisiert vorliegt.

10. Verfahren nach einem der vorhergehenden Ansprüche,
**dadurch gekennzeichnet, dass**
die Lektine so ausgewählt werden, dass sie mit einer Zuckerstruktur des rekombinanten Proteins spezifisch wechselwirken.

11. Verfahren nach einem der vorhergehenden Ansprüche, **dadurch gekennzeichnet, dass**
die rekombinanten Proteine ohne eine Markierung eingesetzt werden.

12. Verfahren nach einem der vorhergehenden Ansprüche
**dadurch gekennzeichnet, dass**
das Verfahren die folgenden Schritte umfasst:
a) Bindung mindestens eines Lektins auf einer Mikrotiterplatte;
b) In-Kontakt-Bringen des Lektins mit dem zu untersuchenden rekombinanten Protein/Glykoprotein;
c) Inkubieren mit einem markierten Detektor, umfassend einen sekundären Antikörper, Protein A und/oder Fragmenten dieser;
d) Nachweis des Detektors mit Hilfe einer Enzym-Substrat-Reaktion, wobei der Detektor und das Enzym deglykosyliert sind.

13. Zuckerbestimmungskit umfassend mindestens einen deglykosylierten Detektor, ausgewählt aus der Gruppe umfassend Antikörper, Protein A und/oder Fragmente hiervon und ein deglykosyliertes Enzym, welches in einer Enzym-Substrat-Reaktion zur Detektion der Wechselwirkung von Proteinen verwendbar ist und gegebenenfalls eine Information zum Kombinieren der Inhalte des Kits.

14. Verwendung des Kits nach Anspruch 12 zur Bestimmung der Zuckerstrukturen von rekombinanten therapeutischen Proteinen, insbesondere Immunoglobulinen.
